# FASCICULE DE BREVET EUROPEEN

(11) **EP 1 004 038 B1**
(45) Date de publication et mention de la délivrance du brevet: **16.10.2002**
(21) Numéro de dépôt: 98925725.8
(22) Date de dépôt: 15.05.1998
(51) Int. Cl.: G01T 1/161, G01T 1/29

(54) **DISPOSITIF DE RADIOGRAPHIE NUMERIQUE A PROTECTION CONTRE LES RISQUES ELECTRIQUES**
DIGITALES RÖNTGENAUFNAHMEGERÄT MIT SCHUTZ GEGEN DIE GEFAHR EINES ELEKTROSCHOCKS
DIGITAL RADIOGRAPHY DEVICE PROTECTED AGAINST RISK OF ELECTROCUTION

(30) Priorité: 16.05.1997 FR 9706049
(43) Date de publication de la demande: 31.05.2000
(73) Titulaire: COMMISSARIAT A L'ENERGIE ATOMIQUE, 75015 Paris Cédex 15 (FR)
(72) Inventeur: THEVENIN, Bernard, F-38120 Saint-Egrève (FR); GLASSER, Francis, F-38320 Eybens (FR)
(74) Mandataire: Poulin, Gérard
(86) Numéro de dépôt international: FR9800977
(87) Numéro de publication internationale: WO98053340

(56) Documents cités:
- EP-A- 0 544 974
- EP-A- 0 666 483
- US-A- 5 434 418

## Description

### Domaine technique

La présente invention concerne un dispositif de radiographie numérique à protection contre les risques électriques.

Ce dispositif peut, avantageusement, être utilisé dans le domaine de la radiographie numérique dentaire.

### Etat de la technique antérieure

La présente invention se situe dans le domaine médical et concerne tout dispositif qui met en relation un appareillage de diagnostic ou de traitement, avec un système d'acquisition, pilotage et/ou traitement, faisant appel au « secteur » pour son alimentation électrique ou à des alimentations d'un autre type dont la tension dépasse les « basses tensions de sécurité ».

Un tel raccordement d'un des éléments de l'appareillage au réseau ne doit occasionner aucun risque pour le patient, l'opérateur et/ou des tiers. Différentes normes font obligation au constructeur de passer en revue tous les risques que peut présenter son appareillage vis-à-vis de ceux-ci et d'y porter remède préventivement, dès l'étude et la construction, en prenant les mesures appropriées de nature à palier aux risques identifiés.

Les dispositifs médicaux de radiographie numérique qui existent actuellement sur le marché, prennent en compte les risques électriques d'une manière classique. Le plus souvent, on place sur le trajet capteur/ordinateur, une « boîte relais », dont la fonction essentielle est d'établir une barrière d'isolement sur le trajet capteur/ordinateur, ainsi que vis-à-vis du secteur qui fournit l'énergie. Cette boîte relais comporte une alimentation secteur à double isolement, qui satisfait aux normes en vigueur, et fournit l'énergie à une électronique locale ainsi qu'au capteur déporté. Cette électronique locale transforme les signaux analogiques provenant du capteur en signaux numériques, de telle sorte que tout échange avec le calculateur ne porte que sur des signaux numériques, qui transitent par des couplages optiques (optocoupleurs) isolant au plan électrique le capteur du calculateur. Ainsi, le capteur est isolé du secteur au niveau de la boîte relais par une alimentation comportant un transformateur à double isolement et, au niveau de l'ordinateur, par la présence sur le trajet des données, d'une barrière optique.

Une telle technique augmente de façon importante le coût induit pour la protection contre les risques électriques et introduit un deuxième élément à risque, à savoir une alimentation indépendante, sans pour autant éliminer l'ordinateur dont il faut également se protéger.

L'invention a pour objet un dispositif permettant de se prémunir contre les risques électriques que l'on peut craindre, lorsqu'un des éléments qui le constituent s'alimente en énergie sur le secteur, ou sur toute autre source dont les tensions sont supérieures à la basse tension de sécurité.

### Exposé de l'invention

La présente invention propose un dispositif de radiographie numérique à protection contre les risques électriques comportant :
- une source de rayonnement, par exemple de rayonnement X ;
- un capteur relié à une carte électronique d'un ordinateur,
caractérisé en ce qu'il comprend un premier et un second ensembles isolés l'un de l'autre du point de vue galvanique, le premier ensemble comprenant des moyens électroniques liés au capteur, le second ensemble comprenant des moyens pour fournir une tension pour alimenter le capteur pendant la présence du rayonnement et une tension pour alimenter la partie capteur de la carte, et des moyens pour assurer la gestion du capteur, la récupération des données et leur traitement, les deux ensembles échangeant des informations par des moyens optiques, et en ce que les deux ensembles cohabitent au sein de la carte électronique.

Avantageusement les deux ensembles sont entourés d'une ceinture d'air.

Avantageusement le premier ensemble comprend une électronique implantée sur la carte électronique et disposée dans un caisson d'isolement galvanique situé sur ladite carte, le capteur et un câble de liaison reliant la capteur et ladite électronique.

Avantageusement l'électronique de l'ensemble capteur est protégée par une ceinture isolante, la liaison caisson d'isolement-câble de liaison se faisant par une prise mobile traversant l'enceinte métallique de l'ordinateur, cette prise disposant d'un isolement renforcé par surmoulage, l'embase fixée du connecteur qui reçoit la prise étant située en retrait dans le caisson d'isolement. Les données analogiques issues du capteur sont numérisées dans l'électronique capteur à l'intérieur du caisson d'isolement.

Avantageusement l'énergie nécessaire à l'ensemble capteur est prélevée dans l'ensemble ordinateur en aval de l'alimentation de sécurité, sur des alimentations basse tension, et transmise par l'intermédiaire de convertisseurs DC/DC situés sur la carte électronique.

Le dispositif de l'invention peut notamment être utilisé dans le domaine de la radiographie dentaire.

Le dispositif de l'invention permet la protection du patient et de l'opérateur contre des risques électriques potentiels mais non certains. Ce dispositif réalise une barrière d'isolement assurant la conformité aux normes électriques entre un capteur, ou tout autre élément, en contact avec un patient à une extrémité et, à l'autre extrémité, une électronique de pilotage et/ou de traitement, alimentée en énergie à partir du secteur.

Le dispositif de l'invention est avantageux du fait de sa simplicité, et donc de son coût, car il met en oeuvre une protection électrique, un traitement des données issues d'un capteur au sein d'une seule et unique carte électronique, et une visualisation de ces données sans sacrifier aucune des exigences élémentaires d'un appareillage de diagnostic.

L'invention peut s'appliquer à tout capteur passif, ou actif, en contact avec un patient dès lors :
- qu'il reçoit, pour son fonctionnement, de l'énergie, éventuellement des stimulis électriques, qui proviennent d'un appareil raccordé au secteur, ou pourvu d'une alimentation autonome, dès lors que sa tension dépasse le niveau de « basse tension de sécurité » (50 Volts) ;
- qu'il fournit, éventuellement, en retour des signaux ou paramètres de mesure, dès lors que ces signaux sont envoyés vers une électronique de traitement alimentée à partir de sources de tension dont la tension dépasse les « basses tensions de sécurité », a fortiori lorsqu'il s'agit du secteur 220 Volts 50 ou 60 Hz.

### Brève description des dessins

- La figure 1 illustre les éléments constitutifs du dispositif de l'invention ;
- les figures 2A et 2B illustrent le dispositif de l'invention en faisant apparaître ses caractéristiques importantes en matière d'isolation, la figure 2B étant une vue de dessus ;
- la figure 3 illustre plus précisément l'agencement de la carte électronique du dispositif de l'invention.

### Exposé détaillé d'un mode de réalisation

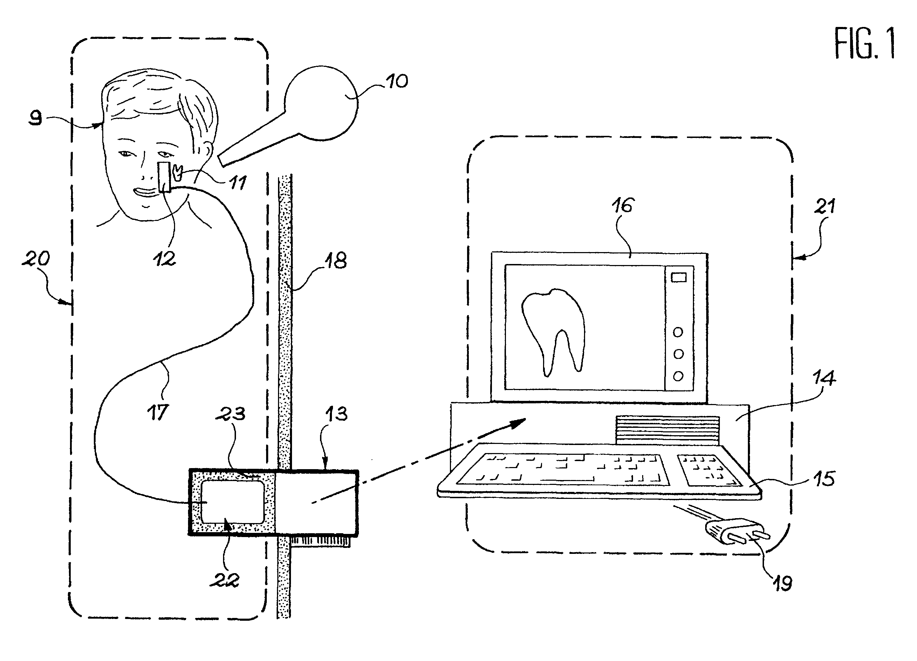

Comme illustré sur la figure 1, le dispositif de radiographie numérique selon l'invention comprend les éléments constitutifs d'un dispositif de radiographie numérique classique, à savoir :
- une source de rayonnement 10 qui émet un rayonnement en direction de l'objet 11 à analyser, ce rayonnement pouvant être un rayonnement X ou γ ;
- au moins un capteur d'images 12 placé derrière l'objet 11, par rapport à la source de rayonnement 10, ce capteur 12 assurant la transformation du rayonnement reçu de la source 10 en informations relatives à la transparence de l'objet 11 au rayonnement ;
- un organe de traitement de ces informations.

L'objet 11 considéré, à titre d'exemple, sur la figure 1 est la dent d'un patient 9, dont on veut faire une radiographie.

L'organe de traitement, représenté sur cette figure, est une carte électronique spécifique 13 d'un ordinateur 14, par exemple de type « Personal Computer », avec son environnement habituel, et notamment un clavier 15, et un écran de visualisation 16. Cette carte 13, représentée à l'extérieur de l'ordinateur 14, sur cette figure, est en réalité à l'intérieur de celui-ci, dans un emplacement standard prévu à cet effet. Elle est reliée au capteur 12 par l'intermédiaire d'un câble de liaison 17.

Cette carte 13 assure simultanément :
- la gestion du capteur 12 sous contrôle de l'ordinateur 14 ;
- la récupération des données de mesure dans le capteur 12 en fin d'examen ;
- la numérisation de ces données, puis leur transfert vers l'ordinateur 14 qui les traite et présente sur l'écran de visualisation 16 l'image radiographique de l'objet 11 considéré.

Le capteur 12, qui est ici un capteur numérique de radiographie dentaire, est associé à un générateur de rayons X 10, mais n'a aucun lien physique ou électrique avec celui-ci. Il remplace le film radiographique utilisé habituellement, qu'il fallait développer. Ce capteur intra-oral actif, pixelisé, constitue, lorsqu'il reçoit un rayonnement X, une image radiographique, traduite en charges électriques au niveau de chaque pixel, qui est visualisée sur l'écran de l'ordinateur 14, une seconde environ après la fin d'émission des rayons X.

Selon l'invention, comme représenté sur les figures 1 et 2A, la carte électronique 13 est scindée en deux ensembles : un ensemble capteur 20 et un ensemble ordinateur 21, qui sont isolés galvaniquement et peuvent tenir une tension d'épreuve conforme aux normes.

Sur la figure 1 est représenté symboliquement une barrière 18 d'isolement entre ces deux ensembles.

L'ensemble capteur 20 comprend d'une part une électronique placée dans un caisson d'isolement galvanique 22 et d'autre part, le câble de liaison 17 qui relie le capteur proprement dit 12 à ce caisson d'isolement.

L'électronique de l'ensemble capteur 20 est protégée par un isolant (époxy 23 ou air 24), de l'ensemble ordinateur 21, réputé à risques du seul fait qu'il tire son énergie 19 du secteur. Cette protection est constituée au minimum, par une ceinture isolante époxy 23 dépourvue de tout conducteur à sa surface comme dans son épaisseur. Cette ceinture isolante jouxte pour une part l'ensemble électronique de l'ordinateur, et permet des échanges sécurisés entre les deux ensembles 20 et 21. Cette ceinture isolante 23, renforcée par des lames d'air 24, assure également l'isolement vis-à-vis de l'enceinte métallique 28 de l'ordinateur.

La liaison caisson d'isolement 22 / capteur 12 se fait par une prise mobile 25 traversant l'enceinte 28 de l'ordinateur 14 avec au droit de la zone de franchissement un isolement renforcé 26 supportant par exemple 4000 volts.

Ce franchissement de l'enceinte 28 par cette prise mobile 25 respecte des dispositions spécifiques de positionnement, de forme et d'isolement, à savoir :
- L'embase fixe 27 du connecteur qui reçoit la prise 25 est située à l'intérieur du caisson d'isolement 22. La frontière de raccordement de la partie mobile sur la partie fixe (embase 27) se fait dans le caisson d' isolement 22 en retrait d'au moins 8 mm de la zone à risques. Ceci n'impose aucune contrainte particulière pour le connecteur utilisé et n'en limite pas le choix.
- La prise mobile 25 comporte un moulage spécial, qui constitue vis-à-vis du câble 17 une isolation renforcée au droit de la zone à risques que représente le franchissement de l'enceinte. Ce moulage est porteur d'un élément de détrompage, ainsi que d'une forme favorisant la saisie et l'extraction.
- Les dispositions adoptées permettent l'emploi d'un connecteur (fiche et embase), ne présentant pas de tenue en tension particulière (500 Volts suffisent).

Les données analogiques issues du capteur 12 (deux paramètres : dose et vidéo) sont numérisées dans l'ensemble capteur 20, à l'intérieur du caisson d'isolement 22, de façon à passer sous forme numérique dans l'ensemble ordinateur, ce qui simplifie ce passage. Les données échangées de façon bidirectionnelle entre les deux ensembles 20 et 21 sont de type numérique et transitent par exemple par des optocoupleurs OPCa, OPCb et OPCc de l'ensemble ordinateur 21 vers l'ensemble capteur 20 et vice versa, ce qui permet de réaliser un isolement galvanique. Ces optocoupleurs ne sont pas bidirectionnels : certains assurent le passage de signaux de l'ensemble ordinateur vers l'ensemble capteur, les autres faisant l'inverse.

L'énergie nécessaire à l'ensemble capteur 20 est prélevée dans l'ensemble ordinateur 21 en aval de l'alimentation de sécurité (alimentation double isolement imposée par les normes), sur des alimentations basse tension (+5V et +12V), et transmise dans l'enceinte de sécurité (ensemble capteur 20), par des convertisseurs DC/DC (DC = courant continu) à haut pouvoir d'isolement (4 000 Volts typique).

Ce mode de réalisation conduit à mettre en série, vis-à-vis du secteur, l'isolement propre de l'alimentation calculateur (déjà renforcée à 3750 Volts) et l'isolement des dispositifs de transfert d'énergie et de signaux (convertisseurs DC/DC et optocoupleurs).

Le concept d' « ensemble » est introduit pour qualifier la réalité d'un isolement galvanique total entre deux régions physiques d'une même carte. Ces deux ensembles électriques cohabitent au sein de la carte 13, mais en aucun cas ne permettent le passage d'un courant électrique d'un ensemble à l'autre. Sur le plan électrique, les deux ensembles 20 et 21 s'ignorent complètement.

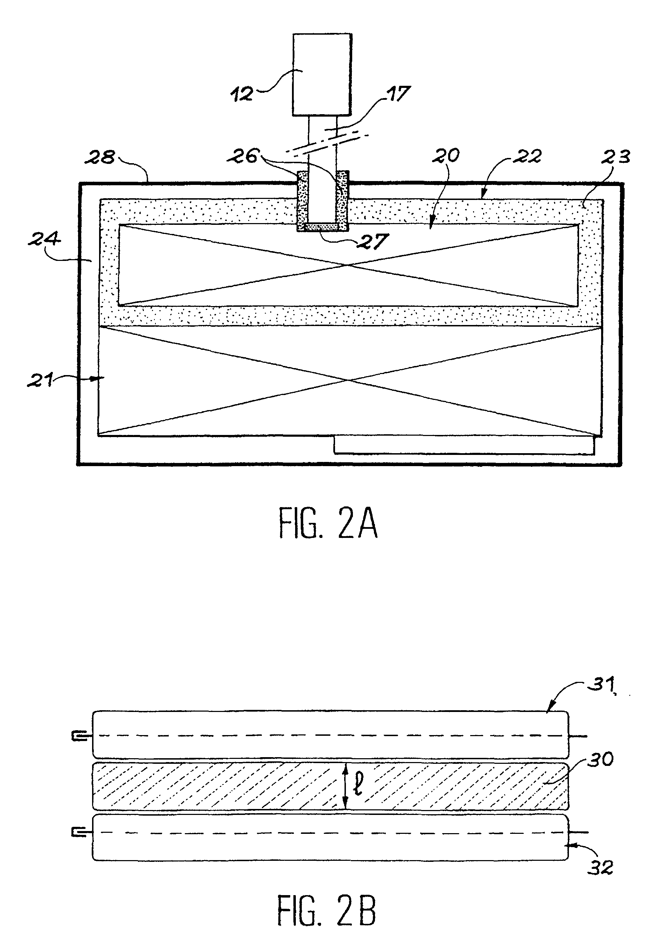

Comme illustré sur la figure 2B, par le biais de l'espacement 1 entre deux cartes 31 et 32, c'est un volume protégé qui est inclus dans l'ensemble ordinateur (lame d'air 30 entre deux cartes voisines, l'une d'elles pouvant être la carte 13).

L'ensemble capteur 20 comprend donc :
- l'électronique implantée sur la carte 13 dans le caisson d'isolement 23 ;
- l'ensemble monobloc connecteur 25, câble 17, capteur 12. Le connecteur 25 permet la mise en relation de cette électronique capteur avec l'application (le patient), via le câble 17 (par exemple de quatre mètres de long).

L'ensemble ordinateur comprend :
- une enceinte métallique 28 ;
- un bus par lequel le processeur exerce son contrôle sur le dispositif, envoie ou reçoit des données ;
- une électronique implantée sur la carte 13 en relation directe avec le bus (bus I.S.A) alimentée par les alimentations +5V et +12V de l'ordinateur.

Tout ce qui isole l'ensemble ordinateur 21 de l'ensemble capteur 20 est prévu pour subir une tension d'épreuve de 4 000 volts. Par contre, l'ensemble câble de liaison 17/capteur 12 qui ne comporte aucune tension supérieure à 50 volts CC, est soumis à une obligation d'isolement réduite à 500 volts.

L'isolement du caisson 22 vis-à-vis de l'électronique ordinateur 21 est assuré au minimum par l'époxy 23 du circuit imprimé dépourvu de tout conducteur dans une zone de 8 mm environ.

L'isolement de ce même caisson 22 vis-à-vis de l'enceinte 28 de l'ordinateur comporte le même isolement époxy, avec en plus une lame d'air de plusieurs millimètres, voire plusieurs centimètres.

L'isolement du caisson 22 sur la carte 13, vis-à-vis de cartes voisines est assuré par une lame d'air 30 garantie par l'écartement entre les connecteurs de ces cartes, qui est de l'ordre de 20 mm (écartement réglementaire nécessaire 4 mm). C'est donc en réalité un volume qui est protégé.

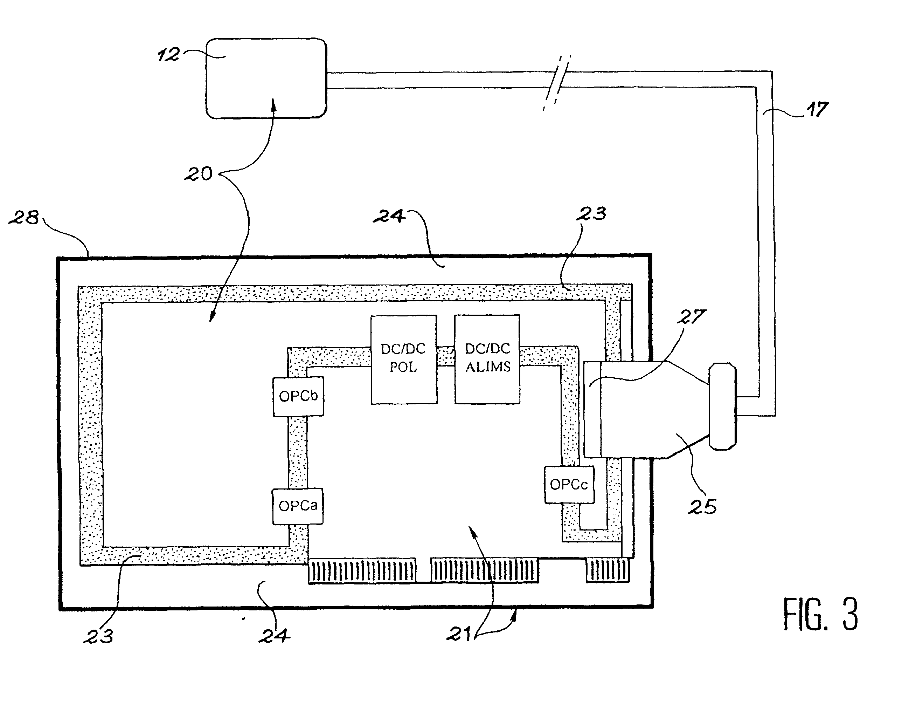

Les convertisseurs DC/DC (DC/DC POL et DC/DC ALIMS), illustrés sur la figure 3, remplissent une double mission :
- Le passage d'énergie d'un ensemble à l'autre se fait avec un isolement galvanique. L'énergie d'électrique devient magnétique grâce à un primaire de transformateur, puis de magnétique redevient électrique sur un ou plusieurs enroulements secondaires, qui sont isolés en fonction des contraintes spécifiées.
- L'énergie prélevée dans un ensemble peut être rendue sur une ou plusieurs sources de tension, ayant le plus souvent des valeurs différentes des valeurs d'entrée.

Pour le convertisseur DC/DC ALIMS, on a en sortie deux sources de tension : +5V et -5V.

Le convertisseur DC/DC POL obéit à une autre loi : sa tension de sortie est de dix fois la tension d'entrée (gamme 2 à 12 volts à l'entrée donnant 20 à 120 volts en sortie) . Il est de plus muni d'une commande logique permettant sa mise en route durant le temps de fonctionnement strictement nécessaire, c'est-à-dire en présence de rayons X (0,1 seconde en typique).

Les deux éléments décrits ci-dessus sont définis par la tension d'isolement supportée entre la source d'entrée et la (ou les) source(s) de sortie, par exemple 4 000 volts.

Si les normes évoluent, le cahier des charges de ces éléments change, les principes d'isolement mis en oeuvre dans le dispositif de l'invention restent valables.

La mise en oeuvre de l'invention telle que définie ci-dessus est rendue possible, grâce aux éléments suivants :
- L'ensemble des besoins en surface de l'électronique appartenant à l'ensemble capteur 20, ainsi que les besoins en surface de l'électronique ordinateur 14, sont réduits de façon drastique par l'emploi d'éléments hautement intégrés, de telle sorte que ces deux ensembles 20 et 21, en se contentant de surfaces très réduites, sont implantables sans difficulté sur une carte électronique.
- La possibilité de traiter les problèmes électroniques et en même temps les problèmes liés à la sécurité sur une carte électronique 13 en apparence semblable à toute autre, par des dispositions simples, constitue un avantage économique important.
- Le caisson d'isolement 22 de sécurité de l'ensemble capteur 20 a une frontière commune avec l'ensemble ordinateur 21 réputé hostile, mais il pourrait avoir en vis-à-vis d'un second monde hostile, constitué par quelques signaux provenant de la source de rayonnement X par exemple. Dans ce cas, les principes exposés restent les mêmes, l'ensemble capteur 20, dans son caisson 22, se protégeant de deux ou de N ensembles réputés hostiles.

Les dispositions prises pour que l'ensemble capteur 20 quitte l'ensemble ordinateur 21 sont également très spécifiques en ce sens que :
- L'embase fixe 27 du connecteur 25 et le plan de connexion/déconnexion sont situés dans le caisson d'isolement 22, en retrait de la face avant de la carte (ensemble ordinateur) et de l'enveloppe mécanique de l'ordinateur (retrait de 8 mm minimum).
- La prise mobile 25 reliant le câble 17 et le capteur 12 à la carte 13, une fois en place, franchit la face avant de la carte 13 et l'enceinte 28, avec au droit de ce franchissement un isolement renforcé, conforme aux normes.

Ces dispositions seraient valables pour toute autre enceinte que celle d'un ordinateur. Il résulte de cette disposition que les contraintes d'isolement ci-dessus ne concernent en rien le connecteur proprement dit qui peut avoir un isolement classique de 500 volts, ce qui ouvre de nombreuses possibilités de choix, tout en limitant le coût.

Il n'est pas présumé de la forme relative de chaque ensemble implanté sur la carte 13, ni des proportions de chacune des surfaces. La frontière peut, par des contours appropriés, être allongée ou réduite selon les besoins d'échange entre les ensembles. Cette frontière peut être établie entre des signaux provenant de l'ordinateur et éventuellement des signaux de provenance externe.

Le principe de l'invention reste valable pour des tensions d'isolement plus élevées en élargissant la barrière époxy à une valeur en rapport avec le besoin, et en utilisant des convertisseurs DC/DC et des opto-coupleurs répondant au nouveau besoin.

Le principe de l'invention reste valable pour toute carte électronique appartenant à d'autres systèmes (mécaniques et électriques par leurs bus : bus VME, VXI, PCI, PCMCIA) présents et à venir, dès lors qu'ils présentent un risque électrique potentiel pour un patient et ou un opérateur.

Le capteur en contact avec le patient peut être de toute autre nature, passif ou actif, dès lors qu'il existe une nécessité de se protéger contre des risques électriques.

Le fait de ne pas avoir recours à une boîte relais, placée sur le câble et chargée de traiter les problèmes d'isolement, présente un autre avantage déterminant : en prenant comme source d'énergie pour alimenter l'ensemble capteur 20, à travers les convertisseurs DC/DC à haut isolement, les alimentations de l'ordinateur 14, on constitue une chaîne de protections en série. En effet, les normes en vigueur imposent à l'alimentation de l'ordinateur un isolement de 3 750 volts et un dispositif comportant un isolement renforcé ou un double isolement. En partant de ces niveaux de tension déjà protégés et en ajoutant une barrière de protection supplémentaire, on double le niveau de protection final.

## Revendications

1. Dispositif de radiographie numérique à protection contre les risques électriques comportant :
- une source de rayonnement (10) ;
- un capteur (12) relié à une carte électronique (13) d'un ordinateur (14) ;
**caractérisé en ce qu'**il comprend deux ensembles (20, 21) isolés l'un de l'autre du point de vue galvanique : le premier ensemble (20) comprenant des moyens électriques liés au capteur (12) et le second ensemble (21) comprenant des moyens pour fournir une tension pour alimenter le capteur (12) pendant la présence du rayonnement et une tension pour alimenter la partie capteur de la carte (13), des moyens pour assurer la gestion du capteur, la récupération des données issues de celui-ci et leur traitement ; les deux ensembles (20, 21) échangeant des informations par des moyens optiques, et **en ce que** les deux ensembles cohabitent au sein de la carte électronique (13).

2. Dispositif selon la revendication 1, dans lequel la source de rayonnement est une source de rayonnement X.

3. Dispositif selon la revendication 1, dans lequel les deux ensembles (20, 21) sur la carte électronique (13) sont entourés d'une ceinture d'air.

4. Dispositif selon la revendication 1, dans lequel le premier ensemble (20) comprend une électronique implantée sur la carte électronique (13) et disposée dans un caisson d'isolement galvanique (22) situé sur ladite carte électronique (13), le capteur (12), et un câble de liaison (17) reliant le capteur (12) à cette électronique.

5. Dispositif selon la revendication 4, dans lequel l'électronique de l'ensemble capteur (20) est protégée par une ceinture isolante (23).

6. Dispositif selon la revendication 4, dans lequel la liaison caisson d'isolement (22)/câble de liaison (17) se fait par une prise mobile (25) traversant l'enceinte métallique (28) de l'ordinateur (14), cette prise disposant d'un isolement renforcé par surmoulage.

7. Dispositif selon la revendication 6, dans lequel l'embase fixe (27) du connecteur, qui reçoit la prise (25), est située en retrait dans le caisson d'isolement (22).

8. Dispositif selon la revendication 4, dans lequel les données analogiques issues du capteur (12) sont numérisées dans l'électronique capteur à l'intérieur du caisson d'isolement (22).

9. Dispositif selon la revendication 1, dans lequel l'énergie nécessaire à l'ensemble capteur (20) est prélevée dans l'ensemble ordinateur (21) en aval de l'alimentation de sécurité, sur les alimentions basse tension, et transmise par l'intermédiaire de convertisseurs continu/continu situés sur la carte électronique (13).

10. Utilisation du dispositif selon l'une quelconque des revendications précédentes, dans le domaine de la radiographie dentaire.

## Patentansprüche

1. Digitales Radiographiegerät mit Schutz gegen die elektrischen Gefahren, umfassend:
- eine Strahlungsquelle (10);
- einen Sensor (12), verbunden mit einer Elektronikkarte (13) eines Computers (14);
**dadurch gekennzeichnet,**
**dass** es zwei voneinander galvanisch isolierte Einheiten (20, 21) umfasst: die erste Einheit (20) mit elektrischen Einrichtungen, die mit dem Sensor (12) verbunden sind, und die zweite Einheit (21) mit Einrichtungen, die eine Spannung liefern, um den Sensor (12) während der Präsenz der Strahlung zu versorgen, und eine Spannung, um den Sensorteil der Karte (13) zu versorgen, sowie Einrichtungen zur Steuerung bzw. Kontrolle des Sensors und zur Erfassung und Verarbeitung der von ihm gelieferten Daten, wobei die beiden Einheiten (20, 21) Informationen bzw. Daten mittels optischer Einrichtungen austauschen,
und dadurch, dass beide Einheiten zusammen auf der Elektronikkarte (13) vorgesehen sind.

2. Gerät nach Anspruch 1, bei dem die Strahlungsquelle eine Röntgenstrahlenquelle ist.

3. Gerät nach Anspruch 1, bei dem die beiden Einheiten (20, 21) auf der Elektronikkarte (13) von einem Luftgürtel umschlossen werden.

4. Gerät nach Anspruch 1, bei dem die erste Einheit (20) eine Elektronik umfasst, vorgesehen auf der Elektronikkarte (13) und untergebracht in einem galvanisch isolierenden Gehäuse (22), das auf dieser Elektronikkarte (13) angeordnet ist, wobei der Sensor (12) durch ein Verbindungskabel (17) mit dieser Elektronik verbunden ist.

5. Gerät nach Anspruch 4, bei dem die Elektronik der Sensoreinheit (20) durch einen Isolationsgürtel (23) geschützt wird.

6. Gerät nach Anspruch 4, bei dem die Verbindung zwischen Isolationsgehäuse (22) und Verbindungskabel (17) durch einen beweglichen Stecker (25) gebildet wird, der das Metallgehäuse (28) des Computers (14) durchquert, wobei dieser Stecker über eine durch Ausgießen verstärkte Isolation verfügt.

7. Gerät nach Anspruch 6, bei dem der feste Sockel (27) des Verbinders, der den Stecker (25) aufnimmt, in dem Isolationsgehäuse (22) zurückversetzt angeordnet ist.

8. Gerät nach Anspruch 4, bei dem die durch den Sensor (12) gelieferten analogen Daten in der Sensorelektronik im innern des Isolationsgehäuses (22) digitalisiert werden.

9. Gerät nach Anspruch 1, bei dem die für die Sensoreinheit (20) benötigte Energie der Computereinheit (21) den Niederspannungsversorgungen entnommen wird, stromabwärts von der Sicherheitsversorgung, und durch Gleichspannungswandler übertragen wird, die sich auf der Elektronikkarte (13) befinden.

10. Verwendung der Vorrichtung nach einem der vorangehenden Ansprüche auf dem Gebiet der Dentalradiographie.

## Claims

1. A digital radiography device with protection against electrical risks, having:
- a radiation source (10);
- a sensor (12) connected to an electronic card (13) of a computer (14);
**characterised in that** it comprises two assemblies (20, 21) isolated from each other from the galvanic point of view: the first assembly (20) comprising electronic means connected to the sensor (12), and the second assembly (21) comprising means for supplying a voltage for supplying the sensor (12) during the presence of radiation and a voltage for supplying the sensor part of the card (13), means for providing management of the sensor, recovery of the data issuing from the latter and processing thereof; the two assemblies (20, 21) exchanging information by optical means, and **in that** the two assemblies cohabit within the electronic card (13).

2. A device according to Claim 1, in which the radiation source is an X-ray source.

3. A device according to Claim 1, in which the two assemblies (20, 21) on the electronic card (13) are surrounded by a belt of air.

4. A device according to Claim 1, in which the first assembly (20) comprises electronics located on the electronic card (13) and disposed in a galvanic isolation enclosure (22) situated on the said electronic card (13), the sensor (12), and a connecting cable (17) connecting the sensor (12) to these electronics.

5. A device according to Claim 4, in which the electronics of the sensor assembly (20) are protected by an isolating belt (23).

6. A device according to Claim 4, in which the connection between isolating enclosure (22) and connecting cable (17) is effected by a movable plug (25) passing through the metallic casing (28) of the computer (14), this plug having insulation increased by superimposed moulding.

7. A device according to Claim 6, in which the fixed base (27) of the connector, which receives the plug (25), is situated recessed in the isolating enclosure (22).

8. A device according to Claim 4, in which the analogue data issuing from the sensor (12) are digitised in the sensor electronics within the isolating enclosure (22).

9. A device according to Claim 1, in which the power necessary for the sensor assembly (20) is taken off in the computer assembly (21) downstream from the safety supply, on the low-voltage supplies, and transmitted by means of DC/DC converters situated on the electronic card (13).

10. Use of the device according to any one of the preceding claims, in the field of dental radiography.
